# EUROPEAN PATENT APPLICATION

(11) **EP 4 227 386 A1**
(43) Date of publication of application: **16.08.2023**
(21) Application number: 21877647.4
(22) Date of filing: 06.10.2021
(51) Int. Cl.: C10L 1/08, C12P 5/00

(54) **METHOD AND SYSTEM FOR PRODUCING DIESEL FUEL HAVING IMPROVED PROPERTY**

(30) Priority: 07.10.2020 JP 2020169481
(71) Applicant: GGI WORLDWIDE MANAGEMENT LTD, JE3 1JS St. Lawrence (JE)
(72) Inventor: NAKAMURA, Hyroyasu, Kanagawa 2380022 (JP); YOSHIKAWA, Naoya, Tokyo 1500031 (JP)
(74) Representative: MacLachlan & Donaldson
(86) International application number: PCT/JP2021/036923
(87) International publication number: WO 2022/075348

(57) **Abstract**

The present invention is for producing a diesel fuel having, as compared to a source oil in a starting material, at least one improved property selected from the group consisting of the cetane rating, cetane index, polycyclic aromatic mass, flash point, sulfur content mass, residual carbon content mass of a 10%-residual oil, pour point, ash content mass, and viscosity. In order to do so, the present invention involves: preparing an aqueous phase by mixing a water soluble alcohol with respect to an enzyme aqueous solution containing 0.01-0.10 mass% of lipase as a first enzyme, so as to obtain an alcohol aqueous solution having a concentration of 20-35%; preparing an oil phase by adding, to the source oil, a second enzyme diesel oil solution obtained by dissolving 0.0001-0.01 mass% of a pineapple-derived enzymes including bromelain in a diesel oil; adding, to the oil phase, the aqueous phase such that the volume ratio of aqueous phase : oil phase becomes 2 to 4 : 8 to 6 while maintaining a temperature lower than 60°C; and mixing the oil phase and the aqueous phase and bringing same into contact with each other through shaking or stirring at normal temperature to cause a reaction between the aqueous phase and the oil phase to improve the property of the oil phase and to produce said diesel fuel.

## Description

### TECHNICAL FIELD

The invention relates to a method for producing diesel fuel. More particularly, the invention relates to a method and a system for producing a diesel oil which satisfy various required standards such as JIS standard and EN standard, and which has improved physical properties in comparison with the starting oil used as a starting material.

### BACKGROUND ARTS

Light oil is generally a petroleum fraction having a boiling point in the range of from about 170 to 370°C, and is used as diesel fuel (hereinafter referred to as Petro diesel), such as fuel for automobile diesel engines, in addition to power generation, agricultural and construction machinery diesel engine fuels, and a fuel for heating such as boilers. In a combustion system where a combustion device, for example, a pyrolysis furnace is used to pyrolyze waste or such to obtain an oil, superheated steam is used as an auxiliary heat source.

Petro diesel is composed of approximately 75% of saturated hydrocarbons (mainly paraffins, including n, iso, and cycloparaffins) and 25% of aromatic hydrocarbons (including naphthalene and alkylbenzenes). Table 1 shows the standards for light oil in Japan, and Table 2 shows the standards in Europe (EN590).

**Table 1**

| **Item** | **Kind** | | | | |
|---|---|---|---|---|---|
| | **S1** | **1** | **2** | **3** | **S3** |
| Flash point °C | 50≥ | 50≥ | 50≥ | 45≥ | 45≥ |
| Distillation Properties of Distillation Temperature (°C) | 360≤ | 360≤ | 350≤ | 330≤ 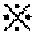 ¹ | 330≤ |
| Pour Point (°C) | +5≤ | -2.5≤ | -7.5≤ | -20≤ | -30≤ |
| Plugging Point(°C) | - | -1≤ | -5≤ | -12≤ | -19≤ |
| Carbon Residue % of 10% Residual Oil | 0.1≤ | | | | |
| Cetane Index 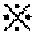 ² | 50≥ | 50≥ | 45≥ | 45≥ | 45≥ |
| Kinematic Viscosity(30°C) mm²/s{cSt} | 2.7≥ | 2.7≥ | 2.5≥ | 2.0≥ | 1.7≥ |
| Sulfur Content% | 0.0010≤ | | | | |
| Density (15°C)**g**/cm³ | 0.86≤ | | | | |

| | | | | | |
|---|---|---|---|---|---|
| 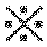 1. When kinematic viscosity (30°C) is not more than 4.7mm²/s{4.7cSt, } distillation properties of distillation temperature is not more than 350°C 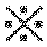 2. Instead of cetane index, cetane value may be used | | | | | |

**Table 2**

| Generally applicable requirements and test methods | | | | |
|---|---|---|---|---|
| Property | Unit | Lower Limit | Upper Limit | Test Method |
| Cetane Index | | 46,0 | - | EN ISO 4264 |
| Cetane Number | | 51,0 | - | EN ISO 5165 |
| Density at 15°C | kg /m³ | 820 | 845 | EN ISO 3675, EN ISO 12185 |
| Polycyclic aromatic hydrocarbons | % (m/m) | - | 11 | EN ISO 12916 |
| Sulphur content | mg / kg | - | 50,0 | EN ISO 20846, EN ISO 20847, EN ISO 20884 |
| | | | 10,0 (on the 01-01-2009) | EN ISO 20846, EN ISO 20884 |
| Flash point | °C | Above 55°C | - | EN ISO 2719 |
| Carbon residue (on 10% distillation residue) | % (m/m) | - | 0,30 | EN ISO 10370 |
| Ash Content | % (m/m) | - | 0,01 | EN ISO 6245 |
| Water Content | mg / kg | - | 200 | EN ISO 12937 |
| Total contamination | mg / kg | - | 24 | EN ISO 12662 |
| Copper strip corrosion (3 hours at 50 °C) | Rating | Class 1 | Class 1 | EN ISO 2160 |
| Oxidation Stability | g/m³ | - | 25 | EN ISO 12205 |
| Lubricity, corrected wear scar diameter (wsd 1.4) at 60°C) | pm | - | 460 | EN ISO 12156-1 |
| Viscosity at 40°C | mm ² / s | 2,00 | 4,50 | EN ISO 3104 |
| Distillation recovered at 250 °C, 350 °C | % V / V | 85 | <65 | EN ISO 3405 |
| 95%(V/V) recovered at | °C | - | 360 | |
| Fatty acid methyl ester content | %(V/V) | - | 7 | EN 14078 |

In addition to Petro diesel, non-petroleum derived alternatives such as biodiesel, BTL, or GTL have been developed. For example, as biodiesel, there are a method for extracting oil by pyrolyzing a carbonaceous raw material previously invented by us, and a method for producing a biodiesel by esterification reaction of oil and fat in the presence of an alcohol utilizing a specific yeast-derived lipase as disclosed in Patent Document 1.

Diesel engines using such light oil are advantageous in obtaining good fuel efficiency and realizing high torque, and have the disadvantage that the exhaust gas contains a large amount of nitrogen oxides (NOₓ) and particulate matter (PM) and, thus, is not clean. Therefore, diesel engines using light oil are subject to various emission regulations.

As an exhaust gas regulation, for example, Euro 5, which will be introduced in the European Union (EU) from 2009, is a stricter exhaust gas regulation for automobiles in consideration of the environment. Euro 5 requests to drastically reduce emission rates, where rate of PM (particulate matter) should reduce 80% (from 0.025g/km to 0.005g/km) and NOₓ (nitrogen oxides) should reduce 20% (from 0.25g/km to 0.2g/km), in comparison with Euro 4. In the EU, Euro 1, which has already been introduced in 1992, required emission limits for diesel and gasoline vehicles. The standards have subsequently moved to Euro 2 (Jan 96), Euro 3 (Jan 2000) and then Euro 4 (Jan 2005). In particular, Euro 4 is a strict requirement to halve PM and NOₓ in diesel passenger cars and to halve CO (carbon monoxide), hydrocarbons and NOₓ in gasoline passenger cars. Euro 5 will introduce even tougher regulations, which will apply to all new vehicles 18 months after entry into force and all newly registered vehicles 36 months after entry into force. Furthermore, in 2014, a transition to Euro 6, which requires diesel vehicles to meet the same emission standards as gasoline vehicles, is scheduled, and **int**erest in hybrid vehicles, bioethanol fuel, hydrogen fuel, and fuel cells has been increased.

As another measure against exhaust gas from diesel fuel, exhaust gas recirculation is used to take in a portion of the exhaust gas after combustion, mainly for the purpose of reducing nitrogen oxides (NOₓ) in the exhaust gas and improving fuel efficiency at partial load, and re-inhaling it. (Exhaust Gas Recirculation: EGR) system has been proposed.

In addition, as another measure against exhaust gas from diesel fuel, a device for simultaneously removing carbon (CO) and nitrogen oxides (NOₓ) by a three-way catalyst (Three-Way Catalyst, TWC) supporting platinum, palladium, and rhodium.

Furthermore, a selective catalytic reduction (SCR), which converts NOₓ into nitrogen molecules N₂ and water H₂O with a catalyst, has also been proposed as a NOₓ purification device. In particular, an exhaust gas purification system that combines EGR and urea SCR using a urea-based catalyst has been adopted by some major automobile manufacturers, but due to the high cost, other automobile manufacturers are reluctant to adopt it. As a result, it developed into a scandal of camouflaged exhaust gas.

In this way, in addition to retrofitting a diesel engine, Patent Document 2 (Japanese Patent No. 5338268) proposes a low compression ratio clean diesel engine that employs EGR without using a NOₓ catalyst.

As an attempt different from devising the diesel engine side, an attempt to reform the diesel fuel itself has also been made.

In the 1990s, oil supply companies launched premium light oil as gas oil with higher added value than general light oil (normal light oil). Compared to ordinary light oil (normal light oil), to this premium light oil are added a "cleaning agent" that removes dirt from the fuel injection system, and a cetane number improver (cetane number + 3) that improves self-ignition (ignition) and reduces white smoke at low temperature start and black smoke at high load. degree), and rust inhibitors (Patent Document 3: JPA-H5-132682).

The composition of Patent Document 3 contains 2 to 15 parts of a low-temperature fluidity improver (ethylene-vinyl acetate copolymer-based additive) and 5 to 25 parts of a cetane number improver (alkyl nitrate having 6 or 8 carbon atoms), and 1 to 10 parts of a detergent (imide additive) for preventing contamination of the fuel injection nozzle, where the cetane number improver is said to be mainly due to the generation of nitro radicals.

In addition, the premium light oil produced by these companies disappeared from the market after 2012 due to reasons such as high cost.

Patent Document 4 discloses a method for producing fuel oil by allowing enzyme water prepared by mixing natural plant complex enzymes with water to be reacted with petroleum oil. According to this method, fuel efficiency can be improved, generation of harmful substances can be easily suppressed, and it is described as being stable.

### PRIOR ART DOCUMENTS

### Patent Documents

Patent Document 1: JPA-2002-233393
Patent Document 2: Japanese Patent No. 5338268
Patent Document 3: JPA-H5-132682
Patent Document 4: Japanese Patent No. 4397432

### SUMMARY OF THE INVENTION

### PROBLEMS SOLVED BY THE INVENTION

However, the method according to Patent Document 4 is a method of producing fuel oil from each petroleum oil, and when this is applied to light oil fuel, the reaction time is very long, and the resultant fuel sometimes deviates from standard of Japan, Europe, etc. Also, it is difficult for this method to obtain diesel fuel with stable properties. The performance of the resultant oil varies depending on the properties of the base oil used as the starting material. Furthermore, similar to general diesel fuel, Patent Document 4 cannot apply the problems that suppress exhaust gas such as NOₓ, SOₓ, CO, PM, etc. to make clean exhaust gas, while cleaning exhaust and that improves fuel efficiency by improving combustion efficiency.

There is a demand for diesel fuel that meet the standards of almost all countries and that can be used with almost all diesel engines (diesel engines) while taking advantage of the advantages of high fuel efficiency and high torque while suppressing NOx and PM and emitting clean exhaust gas.

Therefore, an object of the present invention is to provide a method for efficiently producing diesel fuel that meets the standards of each country and has improved properties compared to the starting material, using Petro diesel-derived light oil as the starting material.

Another object of the present invention is to provide a system for efficiently producing diesel fuel that meets the standards of each country and has improved properties compared to the starting material, using light oil derived from Petro diesel as the starting material.

### MEANS FOR SOLVING PROBLEMS

The present invention, which solves the above object, relates to the following Items.
1 A method for producing diesel fuel having improved properties at least one property selected from the group consisting of a cetane number, a cetane index, a polycyclic aromatic content, a flash point, a sulfur content, carbon residue on 10% distillation residue, a pour point, ash weight and viscosity using light oil derived from Petro diesel, comprising the following stages:
   (a) a water phase preparation stage for preparing a water phase which comprises mixing an aqueous enzyme solution containing 0.01 to 0.1 % by weight of a lipase enzyme as a first enzyme with a water-soluble alcohol so as to be an aqueous alcohol solution having an alcohol concentration of from 20 to 35%;
   (b) an oil phase preparation stage for preparing an oil phase which comprises adding a second enzyme solution in light oil having 0.001 to 0.01% by weight of pineapple-derived enzyme containing bromelain, dissolved in light oil to said base oil;
   (c) a stage for mixing the oil phase with the water phase which comprises adding the water phase prepared in stage (a) to the oil phase prepared in stage (b) with stirring, while maintaining the temperature not exceeding 60°C, to be the volume ratio of the water phase to the oil phase to within the range of 2-4: 8-6;
   (d) a reaction stage, which comprises leaving the oil/water mixture at normal room temperature while continuously or discontinuously contacting both phases with vibration or stirring to cause the reaction between the water phase and the oil phase to improve the properties of the oil phase and to produce diesel fuel; and
   (e) a separation and refining stage, which comprises, after the reaction stage (d), separating the oil phase from the water phase, and refining the separated oil phase by precision filtration.
2 The method according to Item 1, wherein the stage (c) for mixing the oil phase with the water phase comprises adding the water phase to the oil phase at a pressure of 2.5-3MPa and at a liquid temperature not exceeding 40°C.
3 The method according to Item 1 or 2, wherein the stirring is conducted in vortex flow.
4 The method according to anyone of Items 1 to 3, which further comprises, after the stage (c) for mixing the oil phase with the water phase, a stage for removing impurities which comprises applying said oil water mixture to a pulse wave or vibration to remove impurities from the oil phase
5 The method according to anyone of Items 1 to 4, wherein the water phase is prepared by using soft water having a hardness in the range of 0-60mg/L as a water source of said aqueous enzyme solution, adding said first enzyme to the soft water, and maturing the enzyme at a temperature of from 20 to 30°C with aeration over a period of at least 60 hours to prepare the aqueous enzyme solution, and then adding the alcohol to the prepared aqueous enzyme solution.
6 The method according to Item 3, wherein said soft water is modified by one or multiple set of treatment of radical treatment and radical scavenge treatment.
7 Diesel fuel having improved properties produced by the method according to anyone of Items 1 to 6.
8 A system for producing diesel fuel having improved properties produced by the method according to anyone of Items 1 to 6, comprising:
   A1: an water phase line having a water phase tank which stores the water phase prepared by mixing the aqueous enzyme solution containing 0.01-0.10% by weight of lipase as the first enzyme with the water-soluble alcohol so as to be an aqueous alcohol solution having an alcohol concentration of from 20 to 35%, with aeration;
   B1: a base oil tank which stores a light oil derived from Petro diesel;
   B2: an oil phase line having an oil phase preparation member which prepares the oil phase by adding the second enzyme to said base oil;
   C1: a oil/water mixing tank which meters the prepared oil phase, and added thereto the water phase at a prescribed pressure so that the temperature liquid is within a prescribed range;
   C2: a reactor having a vibrator which vibrates the oil/water mixture from the oil/water mixing tank and/or a pulsing device which pulses the oil/water mixture and refining the separated diesel fuel;
   C3: a separation/refining device which separates diesel fuel having improved properties obtained by the reaction from the water phase; and
   C4: a diesel fuel production line having a storage tank which stores the diesel fuel having improved properties thus separated and purified.
9 The system according to Item 8, which further possesses at least one reactor tank possessing or not possessing a vibrator and/or a pulsing device, on downstream of said reactor
10 The system according to Item 8 or 9, wherein said diesel fuel production line is accommodated within a mobile body.
11 The system according to Item 9, wherein said water phase line further possesses an enzyme mixing tank which admixes raw water from water source with the lipase enzyme as the first enzyme, a maturation tank having an aeration device, which maturates the mixed solution of said raw water with the lipase enzyme with aeration to prepare the aqueous enzyme solution, and a mixing tank which mixes the aqueous enzyme solution thus prepared with the alcohol to prepare the water phase.
12 Use of pineapple-derived enzyme containing bromelain as a reaction accelerator for adding an oil phase, in the course of the production of diesel fuel by admixing a water phase comprising an aqueous alcohol solution containing lipase enzyme with the oil phase comprising Petoro diesel or crude Petro diesel.

### EFFECT OF THE INVENTION

According to the present invention, using a base oil made of light oil derived from Petro diesel as a starting material, diesel fuel can be obtained at about 22.5 to 45% by volume of the base oil, i.e., 1.225 to 1.45 times the total volume of the base oil, by reacting a water phase having an aqueous enzyme solution containing a lipase enzyme as a first enzyme admixed with a water-soluble alcohol to form a 20 to 35% aqueous alcohol solution with an oil phase having a pineapple-derived enzyme containing a second enzyme, bromelain, preliminarily added as a reaction accelerator.

The resulting diesel fuel conforms to the performance standards of each country, and has improved properties at least one property selected from the group consisting of a cetane number, a cetane index, a polycyclic aromatic content, a flash point, a sulfur content, carbon residue on 10% distillation residue, a pour point, ash weight and viscosity. More specifically, the cetane number (cetane index) and flash point are increased, and the aromatic content, kinematic viscosity, and sulfur content are decreased, resulting in a high-quality diesel fuel.

Even if the base oil which does not meet the standards of each country is used as a starting material, for example, even if a raw material with a cetane index of about 40 or a raw material with a sulfur content of about 0.5% by weight is used, the resulting diesel fuel meets the standards of each country.

Furthermore, when the resulting diesel fuel with improved properties is applied to a diesel engine, the amount of CO, SOₓ and PM generated decreases without increasing NOₓ in the exhaust gas. This means that while maintaining the advantages of diesel fuel, such as good fuel efficiency and the ability to obtain high torque, it overcomes the drawbacks that NOₓ and PM are easily emitted and that the exhaust gas is not clean. Such advantages can be attained by the present invention for the first time.

Furthermore, according to the present invention, there is provided a system for stably producing diesel fuel having improved properties conforming to the standards of each country.

### BRIEF DESCRIPTION OF THE DRAWINGS

[FIG. 1] is a flowchart showing the stages of the method for producing diesel fuel of the present invention.
[FIG.2] (a) is a flowchart showing the stage for preparing water phase in FIG. 1 and (b) is a flowchart for modifying water in (a).
[FIG.3] is a schematic view showing one example of the system for producing diesel fuel of the present invention.
[FIG.4] (a) and (b) are schematic views each showing embodiments of the diesel fuel production line in the system for producing diesel fuel of the present invention accommodated within a mobile body.
[FIG.5] shows a schematic view showing another example of the system for producing diesel fuel of the present invention.

### EMBODIMENTS FOR CARRYING OUT THE INVENTION

Now, embodiments of the present invention will be described in detail by referring to the attached drawings.

The terms used in the present invention have the following meanings.

"Petro diesel" is generally a petroleum fraction with a boiling range of approximately 170-370°C, and has properties according the standards shown in Table 1 and Table 2.

"Crude Petro diesel" is incompletely refined Petro diesel, and does not satisfy at least one property among the properties shown in able 1 and Table 2. For example, crude Petro diesel contains sulfur in an amount of about 0.5% by weight.

"Conforming to national standards" means that diesel fuel has properties conforming to the standards of the country to be used. For example, diesel fuel (Petro diesel or diesel fuel composed mainly of Petro diesel) conforming to Japanese standard has properties defined in Table 1 and the diesel fuel conforming to EU standard has properties defined in Table 2.

"Improved properties" means that properties defined in country to be used have been improved in comparison with properties of original starting material

The diesel fuel obtainable in the production method of the present invention using Petro diesel or crude Petro diesel as a base oil has improved properties in comparison with the base oil and has properties adapted to the standards of countries.

### (Production Method)

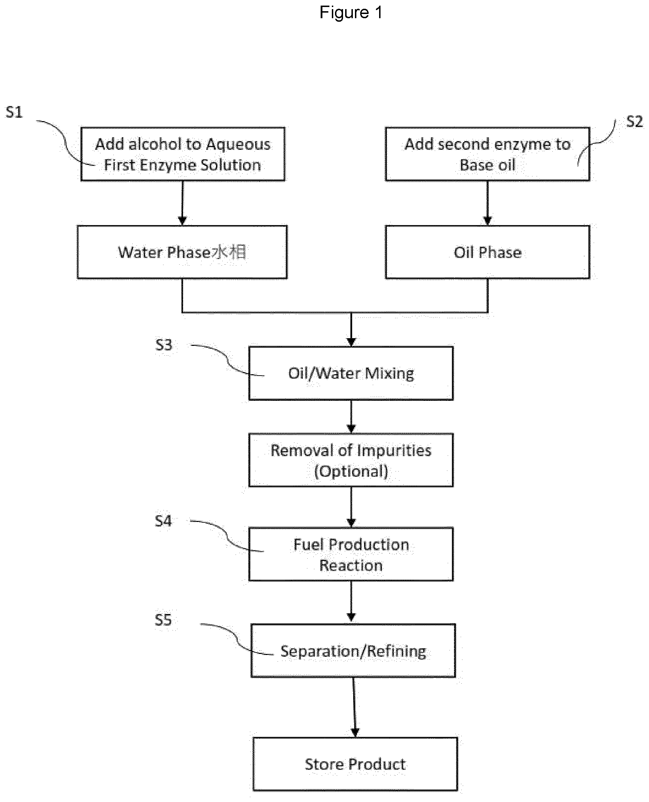

The method of producing diesel fuel of the present invention will now be described by referring to FIG. 1 to FIG.3.

As shown in FIG. 1, the method of producing diesel fuel of the present invention is the method for producing diesel fuel for obtaining (upgraded) diesel fuel conforming to the standards of each country. Specifically, first, an aqueous enzyme solution is prepared and a prescribed amount of alcohol is added to the aqueous enzyme solution thus prepared to prepare a water phase. The water phase is mixed and reacted with an oil phase composed of the raw material of Petro diesel to increase an amount of diesel fuel in the oil phase. The oil phase obtained by separation from the water phase through a precision filter to produce diesel fuel for obtaining (upgraded) diesel fuel conforming to the standards of each country.

In general, there are many reports on the relationship between the properties of diesel fuel and the characteristics of exhaust gas emitted from diesel engines, and cetane number (cetane index), distillation properties, aromatic composition, etc. are cited as major indicators of fuel properties that have a large impact. Whereas the cetane number (cetane index) is closely related to combustion in the engine, so it affects most exhaust gas components, regarding distillation properties, it is said that an increase in heavy content affects particulate matter (PM). It is said that PM emissions increase as aromatic components increase and it is also considered to increase NOₓ emissions. The diesel fuel produced by the production method of the present invention can overcome such contradictory matters, improve fuel properties, and reduce particulate matter (PM) and NOₓ emissions (see Table 3 below) .

The first enzyme used in the present invention is a lipase enzyme or a mixture of a lipase enzyme and a cellulase enzyme, and is used for hydrolysis or catalytic decomposition by stirring and mixing with the oil phase described later and subsequent contact. The first enzyme used in the present invention is not restricted as long as it exhibits the function and effect of the present invention and can be selected from the conventional lipase used in the production of biodiesel and the lipase enzyme described in Patent Document 4. Preference is given to use enzyme "SUPER X" commercially available from GGI Worldwide Management Ltd. This enzyme is an enzyme-containing carrier containing about 70-90% by weight of fruit lipase and about 30-10% by weight of cellulase enzyme carried on a zeolite carrier (hereinafter referred to as "first enzyme").

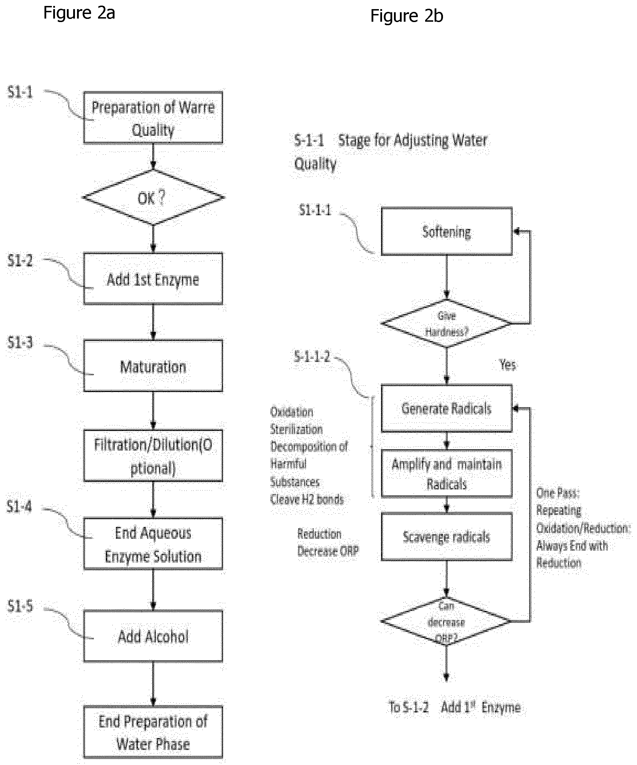

As shown in FIG. 2(a), in the present invention, first, an aqueous enzyme solution of the first enzyme is prepared. The concentration of the enzyme in the aqueous enzyme solution may be adjusted so that the first enzyme is added so as to be 0.01-0.10% by weight of lipase enzyme. Alternatively, the concentration may be adjusted by diluting a concentrated aqueous enzyme solution with water to 0.01-0.10% by weight f lipase enzyme.

The water used for preparing this aqueous enzyme solution is preferably soft water having a hardness of 0 to 60 mg/L. It is not preferable to use water having a hardness exceeding 60 mg/L because minerals such as calcium may inhibit enzyme activity.

When the hardness of raw water to be used is so-called hard water exceeding 60 mg/L, it is preferable to perform so-called water softening to make the hardness less than 60 mg/L, preferably less than 30 mg/L. Such water softening is generally performed by a water softening device using a conventionally well-known water filter for water softening.

In addition to water softening, preferred embodiment of the present invention modifies the raw water to be used. Modification applicable to the present invention includes, for example, modification that uses magnetism to break hydrogen bonds in water molecules.

According preferred embodiment of the present invention, as shown in FIG.2, radicals are applied to water, radicals thus applied are amplified and maintained in the water, and the radials are scavenged. These operations are conducted once or repeatedly.

According to the present inventors, it has been understood that when soft water is passed through an apparatus that repeats the radical generation process, the radical amplification/maintenance process, and the radical scavenge process, microbes in the water are killed, the permeability increases, and the redox potential decreases. It has been found that when the first enzyme is added and matured in such a water environment, the diffusion maturation speed and activation of the first enzyme increase, and the maturation time is shortened by about 10% to 20%. Such an apparatus is disclosed for example in our patent publication JPA2008-15506.

In the present invention, after adding 0.01 to 0.10% by weight of the first enzyme to raw water that has optionally been softened so as to have a hardness of 60 mg/L or less, and modified, the enzymes are activated by maturing for at least 60 hours, preferably 72 hours, with aeration at a temperature of 20-30°C (room temperature). By maturing under such conditions, the effect of the present invention, that is, enzyme water for the aqueous phase is prepared for reforming and producing diesel fuel that meets the increased amount of base oil and the standards of each country. Whereas maturing for at least 72 hours is required if soft water with a predetermined hardness is used, as shown in FIG. 2(b), if water obtained by modifying soft water with a predetermined hardness is used as a raw material, the maturation time can be shortened, for example, the aging time required from 72 hours or more can be shortened to 60 hours.

In addition, when using an enzyme caried on a zeolite carrier as the first enzyme as in this embodiment, the used zeolite is preferably removed by a filter not so as to be mixed with the oil phase
so that the used zeolite does not mix with the oil phase. of the zeolite carrier is removed by a filter, before mixing with the oil phase, preferably at the time of finishing maturation..

In a specific embodiment of the present invention, other enzymes, such as lipase enzymes of different origins and cellulase, can be added prior to or during the maturation process as an auxiliary.

A water phase is prepared by adding and mixing a water-soluble alcohol to the aqueous enzyme solution thus prepared so as to be a 20 to 35% alcohol aqueous solution. Alcohols which can be used herein are not particularly limited as long as they are water-soluble alcohols, and examples thereof include methanol, ethanol (including bioethanol), n-propanol, i-propanol, and mixtures thereof. Methanol is preferred due to its availability and low cost.

On the other hand, the (crude) Petro diesel, which constitutes the oil phase in the present invention and is the base oil, is a petroleum fraction of 170 to 370°C as described above, and contains about 75% saturated hydrocarbons (mainly n-, iso-, and paraffins, including cycloparaffins), and 25% aromatic hydrocarbons (including naphthalene and alkylbenzene), with impurities such as sulfur, nitrogen compounds, metals, and high-boiling compounds such as asphaltenes.

As described in Patent Document 4, if this is simply reacted with the first enzyme alone, the properties of the diesel fuel obtained will not be constant even if the reaction is performed under the same conditions using the same base oil. In some cases, it became a diesel fuel that did not meet the standards of each country.

This is because Patent Document 4 is mainly aimed at reforming heavy oil and increasing the amount of fuel, and does not consider manufacturing fuel that meets the standards of each country.

When we have made extensive studies so that diesel fuel that meets the standards of each country obtained as a whole while modifying the base oil can be reproduced with good reproducibility, it has been found to be solved by adding a second enzyme dissolved in light oil, and the present invention has been created based on such a technical idea.

This second enzyme is marketed as SUPER X by GGI Worldwide Management Ltd. as a cetane improver for diesel fuel. According to our experiments, when a small amount of this enzyme, which is an agent serving as cetane improver, is added to the oil phase, the startability is improved, especially at low temperatures, the cetane number is increased, the combustion efficiency is increased, and the generation of PM is reduced.

In the present invention, the aqueous phase, which is an aqueous alcohol solution containing a predetermined amount of the first enzyme, and the oil phase containing a predetermined amount of the second enzyme are stirred and mixed to bring the first enzyme in the aqueous phase into contact directly or through the interface between the aqueous phase and the oil phase, resulting in the reforming of the (crude) diesel fuel, which is the base oil, and the weight-enhancing reaction that produces the light oil component.

According to the present invention, the action of the second enzyme improves the fluidity, improves the distillation properties and reduces the aromatic content, increases the reactivity of the oil phase, and improves the properties of the resulting diesel fuel. Specifically, by increasing the fluidity, the degree of contact between the water phase and the interface is increased by applying a pulse wave to the oil phase or shaking the oil phase. In addition, the reactivity of the water phase with the alcohol is enhanced by ring-opening of the aromatic component and reduction of the molecular weight of the diesel oil. Therefore, compared with the case where the second enzyme is not added to the oil phase, it is possible to increase the yield (10 to 20%) and shorten the reaction time (10% to about 20%). In addition, although the reason is unknown, as a result of repeated experiments by the present inventors, when the diesel fuel with improved properties obtained by the production method of the present invention is applied to a diesel engine, it is possible to reduce the emissions of carbon monoxide, particulate matter PM, etc. while maintaining the emissions of nitrogen oxides in the exhaust gas within a certain range without increasing the emissions.

At this time, the volume ratio of the aqueous phase to the oil phase is 2 to 4:8 to 6, preferably 2.5 to 3.5:7.5 to 6.5, more preferably about 7:3. If the amount of the aqueous phase is more than the following range, it is disadvantageous in terms of water treatment after the reaction and the cost of the alcohol content of the reaction. Conversely, when the amount is less than the above range, the reaction becomes insufficient.

The method of mixing the water phase and the oil phase is to add the water phase into the oil phase at a room temperature (about 25°C) at a pressure of 2.5 to 3 MPa in a manner that the liquid temperature is kept at a temperature not deactivating the first enzyme (and the second enzyme), i.e., a temperature not exceeding 60°C, preferably not exceeding 45°C and the mixture is stirred similarly while keeping at a temperature not exceeding 60°C, preferably not exceeding 45°C. The reason why the liquid temperature is kept below 60°C is that if the temperature exceeds 60°C, the first enzyme may be deactivated.

The method of stirring may be mixing with a blade, but it is preferable to stir by generating a vortex flow from the viewpoint of not generating frictional heat.

After stirring and mixing the oil phase and the water phase in this manner, if desired, the oil-water mixture is subjected to pulse waves by a pulse generator or vibrated by a vibration device to remove residual impurities. Solids and metals are separated and removed via the aqueous phase.

Continuously or intermittently, the oil phase and the aqueous phase are brought into contact with each other by shaking or stirring, and the mixture is allowed to stand at room temperature for several hours, preferably 20 hours or more, more preferably 40 hours or more, to allow reforming and reaction to proceed.

By allowing to stand in this manner, the first enzyme permeates from the water phase to the oil phase from the interface between the oil phase and the water phase, and hydrolyzes and catalytically cracks the hydrocarbons in the raw diesel fuel to produce higher hydrocarbons. As the lower hydrocarbons are formed, lower olefin formation proceeds (reaction from the interface).

On the other hand, due to the action of the second enzyme added to the oil phase, the aromatics in the oil phase are olefinized and successively converted to lower olefins, and elements such as N and S in the aromatic are removed during oil-water separation, via the aqueous phase during oil-water separation.

In this way, both the action of the first enzyme from the oil-water interface and the action of the second enzyme in the oil phase promote lower olefination and produce an upgraded diesel fuel that meets the standards of each country.

The diesel fuel reformed and manufactured in this way has a higher cetane number (cetane index) and flash point, and a lower aromatic content, kinematic viscosity, and sulfur content, resulting in a higher quality diesel fuel.

Even if the base oil is a diesel fuel that does not conform to the standards of each country, for example, even if a raw material with a cetane index of about 40 or a sulfur content of about 0.5% by weight is used, diesel that meets the standards of each country can be obtained. In the present invention, diesel fuel can be obtained in an amount of about 22.5 to 45 vol relative to 1 volume of the base oil.

As an example, using light oil having the properties shown in Table 3 as a base oil, Table 3 shows properties of diesel fuel produced by the production method of the present invention (fuel of the present invention in the table) and exhaust gas components when Caterpillar 3304 (4 cylinders, total displacement of 7000cc, pre-combustion chamber, 62.5 kW/1800 rpm) is used in comparison with the base oil.

**[Table 3]**

| | | | | | | |
|---|---|---|---|---|---|---|
| Properties | Base Oil | Inventive Fuel | | NO ₓ Emission (ppm) | Base Oil | Inventive Fuel |
| Density (g/cm3) | 0.856 | 0.841 | | Idle | 227 | 227 |
| Aromatics ( | 37.6 | 32.7 | | High Idle | 259 | 255 |
| Cetane index | 48.6 | 53.4 | | Full Power | 465 | 472 |
| Flash Point | 70 | 78 | | 1700rpm | 381 | 385 |
| 90% Fraction (°C) | 325.0 | 326.0 | | 1800rpm | 659 | 646 |
| Sulfur Content (%) | 0.157 | 0.084 | | | | |
| | | | | | | |
| Fuel Consumption(L/Hr.) ) | Base Oil | Inventive Fuel | | Aromatics (mg/m³) | Base Oil | Inventive Fuel |
| Idle | 1.34 | 1.25 | | Idle | 14.8 | 8.4 |
| High Idle | 9.30 | 9.25 | | High Idle | 42.6 | 44.0 |
| Full Power | 26.91 | 27.04 | | Full Power | 17.2 | 16.6 |
| 1700rpm | 18.47 | 18.20 | | 1700rpm | 8.2 | 0.6 |
| 1800rpm | 10.77 | 10.51 | | 1800rpm | 9.8 | 5.8 |
| | | | | | | |
| CO Emission (ppm) | Base Oil | Inventive Fuel | | Particulate Matter (mg/m³) | Base Oil | Inventive Fuel |
| Idle | 113 | 112 | | | | |
| High Idle | 318 | 276 | | Idle | 5 | 1 |
| Full Power | 641 | 562 | | High Idle | 192 | 218 |
| 1700rpm | 707 | 584 | | Full Power | 128 | 127 |
| 1800rpm | 270 | 224 | | 1700rpm | 212 | 171 |
| | | | | 1800rpm | 119 | 103 |

As shown in Table 3, in the present invention, due to the interaction between the action of the first enzyme from the interface and the action of the second enzyme in the oil phase, the density, aromatic content, distillation properties 90% distillation temperature, sulfur content decrease and cetane index increase due to the interaction between the action of the first enzyme from the interface and the action of the second enzyme in the oil phase.

On the other hand, it is considered that the fuel consumption was improved because the amount of fuel used per unit time was decreased. Also, carbon monoxide emissions and particulate matter emissions were reduced, and nitrogen oxide emissions were comparable. As a result, the diesel fuel produced by the production method of the present invention has improved various properties compared to the base oil used as the starting material, comparing to the base oil. Also, when the diesel fuel produced by the production method of the invention is used in a diesel engine, it can be said that it emits cleaner exhaust gas than the base oil.

Furthermore, the method for producing diesel fuel with improved properties of the present invention improves the fluidity of the base oil by adding a second enzyme to the base oil. Therefore, by applying a pulse or vibration to the oil phase in which the second enzyme is added to the base oil, the contact condition with the water phase becomes better than when the second enzyme is not added. This makes it possible to increase the reaction efficiency, increase the yield (from to 10 20%) and shorten the reaction period.

### (System for Producing Diesel Fuel)

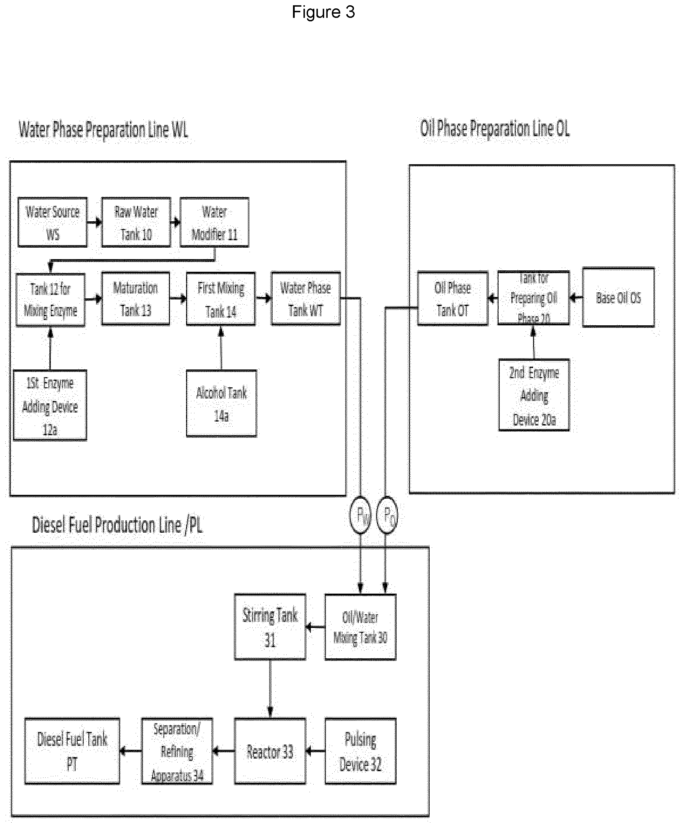

The improved-characterized diesel fuel of the present invention having such excellent properties can be produced, for example, by the system shown in FIGS. 3-5.

Now, a diesel fuel production system for producing diesel fuel by the diesel fuel production method of the present invention (hereinafter referred to as the production system of the present invention) will be described below. In the production system shown below, while the enzyme water is produced without dilution, it is also within the scope of the present invention to prepare a concentrated solution of the first enzyme and dilute it to an aqueous enzyme solution of the desired concentration before use.

The production system of the present invention is mainly comprises an aqueous phase preparation line WL (aqueous phase line A in the claims), an oil phase preparation line OL (oil phase line B in the claims), and a diesel fuel production line PL (diesel fuel production line C in the claims).

The aqueous phase preparation line WL possesses an enzyme mixing tank 12 for adding and mixing raw water from the water source WS and a lipase enzyme as a first enzyme; a maturation tank 13 for preparing an aqueous enzyme solution by maturing the mixed solution of the raw water and the lipase enzyme; and a first mixing tank 14 for preparing an aqueous phase by adding alcohol to the prepared aqueous enzyme solution. When a previously prepared aqueous phase is used, the aqueous phase preparation line WL may be an aqueous phase line composed of an aqueous phase tank in which the aqueous phase is stored while being aerated.

In general, the raw water from the water source WS is stored in the raw water tank 10 and is used after checking whether it meets the raw material standards. Specifically, in the present invention, it is preferable to use soft water having a small amount of minerals such as calcium and magnesium contained in water that inhibit the activity of enzymes.

Generally, soft water is classified as having a hardness of 0 to 100 mg/L, moderately hard water as having a hardness of 101 to 300 mg/L, and hard water as having a hardness of 301 mg/L or more. In the present invention, soft water having a hardness of 60 mg/L or less is preferable.

Therefore, in the present invention, when using water with a high degree of hardness, cations such as calcium ions and magnesium ions contained in the water are reformed by a water softening device (water reforming device 11) having a function of replacing the cations with sodium ions by the action of the ion exchange resin.

When the water from the water source WS, such as tap water, which contains an amount of chlorine and microorganisms more than a predetermined is used as the raw water, it is preferable to modify the water accordingly. It is also within the scope of the present invention to modify the water itself by activating it rather than improving the negative factors contained in the water.

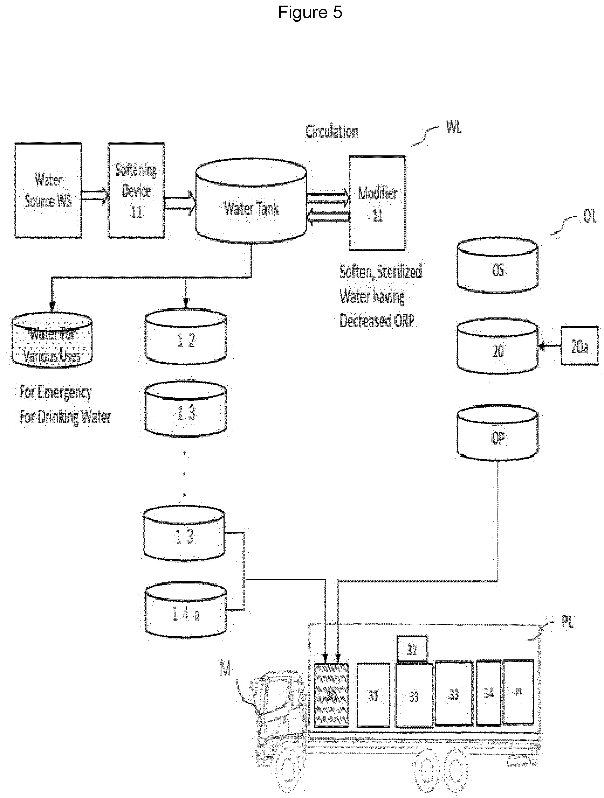

It is preferable to use the aforementioned modification technique shown in FIG. 2(b) (see FIG. 5 for details) as such an apparatus.

In the system of the present invention, a predetermined amount (0.01 to 0.10% by weight of the first enzyme in the case of an undiluted straight enzyme aqueous solution) of the first enzyme is added to the raw water thus modified as desired in the enzyme mixing tank 12. Then, the aqueous solution to which the enzyme has been added in this manner is matured at room temperature for 60 hours (when using a reformer described later) or longer, generally 72 hours or longer, while being aerated by an aerator (not shown) in the maturation tank 13.

In the system of the present invention, it is required for maturation for 60 hours or more, generally 72 hours, so that the maturation tank 13 becomes huge for preparation of a large amount of enzyme solution and production of diesel fuel. For this purpose, this maturation tank 13 can consist of a plurality of tanks 13 arranged in parallel and/or in series. Alternatively, the aqueous enzyme solution may be prepared as a concentrated solution and diluted when mixed with alcohol.

In the system of the present invention, the aqueous enzyme solution thus prepared in the (final) maturation tank 13 is then transferred to the first mixing tank 14, at which a predetermined amount of alcohol is added. At this time, when the first enzyme used is the enzyme carried on the zeolite carrier as described above, the transfer line to the first mixing tank 14 is equipped with a filter for removing the zeolite carrier. Furthermore, as described above, in the first mixing tank, prior to the addition of alcohol, other enzymes, such as lipase enzymes of different origins, cellulase, etc., may be supplementarily added.

Then, the aqueous enzyme solution to which alcohol has been added is preferably stored in the aqueous phase tank WT while being aerated by an aerator (not shown) until it is used as the aqueous phase.

The oil phase preparation line OL is an oil phase preparation line that includes a base oil tank OS for preparing and storing base oil as a raw material, and adding a predetermined amount of the second enzyme to prepare the oil phase. In this embodiment, the oil phase is prepared by adding a predetermined amount of the second enzyme to the predetermined amount of the base oil transferred from the base oil tank OS and introduced into the oil phase preparation tank 20 by the second enzyme addition device 20a. Then, the oil phase prepared in the oil phase preparation tank 20 is transferred to the oil phase tank OT and stored there until it is mixed with the water phase.

In the system of the present invention, the water phase in the water phase tank WT and the oil phase in the oil phase tank OT are separately prepared and stored in the water phase preparation line WL and the oil phase preparation line OL prepared in this manner, and they are mixed and reacted in a diesel fuel production line PL to produce diesel fuel.

The oil-water mixing tank 30 is an tank which first introduces and fills the oil phase in the oil phase tank OT via the pump Po so that the oil phase and the water phase have a predetermined volume ratio, and then pumps the water phase WP to the oil phase thus filled via a pump Pw within a predetermined pressure and temperature range for addition and mixing.

The stirring tank 31 is a tank for stirring the oil/water mixture mixed in the oil/water mixing tank 30. The stirring means at this time can be a blade or the like, but it is preferable to have a stirring device that generates a vortex flow in order to mix the oil and water while controlling heat generation.

The mixed and stirred oil-water mixture is left reacting while keeping the temperature at room temperature for several hours, preferably 24 hours or more, more preferably 48 hours or more, while the oil phase and the water phase are brought into contact with each other by shaking or stirring continuously or intermittently in the reaction tank 34. In a preferred embodiment of the present invention, a pulse imparting device 32 for imparting pulse waves to the oil-water mixture is provided to vibrate and stir the oil-water mixture with the pulse waves.

Since this reaction also requires a long period of time, it is preferable to adopt a configuration in which the reaction is performed in a plurality of reaction tanks 34, preferably the reaction tanks 34 connected in series. Since the reaction of the oil-water mixture sufficiently mixed and stirred in the reaction tank 34 proceeds even if it is left standing, the reaction tank 34 on the downstream side does not need to be provided with a stirring device, a pulse imparting device 32, or the like. When arranging a plurality of reaction tanks 34 in series, it is also possible to adopt a configuration in which a stirring device, a pulse imparting device 32, or the like is provided.

By allowing to stand in this manner, the first enzyme permeates from the water phase to the oil phase from the interface between the oil phase and the water phase, and hydrolyzes and catalytically cracks the hydrocarbons in the raw diesel fuel to convert higher hydrocarbons into lower hydrocarbons whereby lower olefin formation proceeds (reaction from the interface). On the other hand, due to the action of the second enzyme added to the oil phase, the aromatics in the oil phase are olefinized and gradually converted to lower olefins, while the elements such as N and S in aromatics are removed via the aqueous phase during oil-water separation.

In this way, both the action of the first enzyme from the oil-water interface and the action of the second enzyme in the oil phase promote lower olefination and produce an upgraded diesel fuel that meets the standards of each country. The produced diesel fuel is purified after oil-water separation in a refiner and stored in a diesel fuel tank PT, which is a product tank.

Both the action of the first enzyme from the oil-water interface and the action of the second enzyme in the oil phase promote lower olefination to produce an upgraded diesel fuel that meets national standards. The produced diesel fuel is stored in the diesel fuel tank PT, which is the product tank, after oil and water separated in the refiner.

As described above, using an base oil, the system of the present invention reforms the base oil and can reliably produce diesel fuel that can meet the standards of each country in an amount of about 22.5to 45% by volume relative to the base oil, i.e., 1.225 to 1.45 times% the base oil.

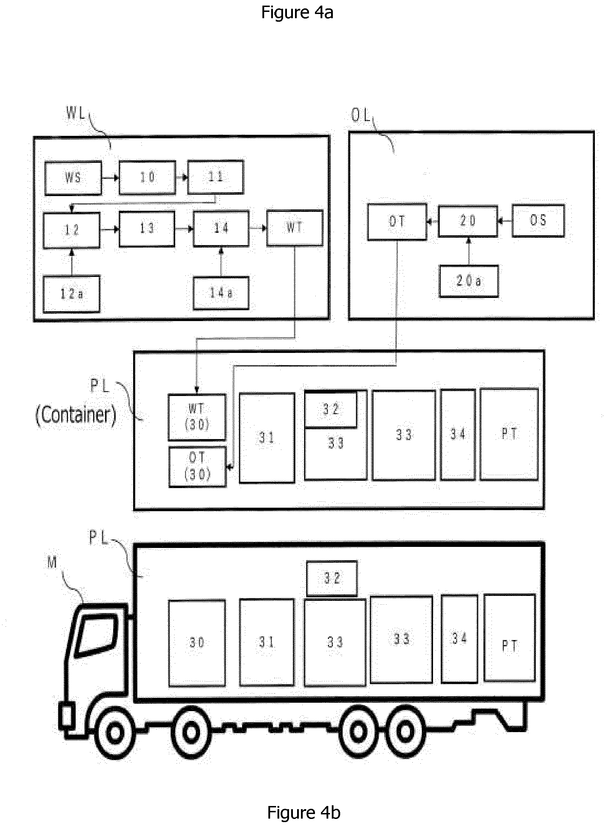

Another embodiment of the system of the present invention will now be described with reference to FIG.4. Components similar to those in FIG. 3 are denoted by the same reference numerals, and descriptions thereof are omitted.

In the system of the present invention, the aqueous phase preparation line WL, which has a large amount of reaction time and installation location, needs to be installed at a fixed installation location, but the oil phase preparation line OL and the diesel fuel production line PL do not necessarily need to be installed at fixed locations. For example, it can be arranged in a transport container shown in FIG. 4(a) and installed in a moving body M such as a truck or ship as shown in FIG. 4(b).

In the embodiment shown in FIG.4, it is possible to install the diesel fuel production line PL on the loading platform of the mobile body M, preferably on a transport container.

By configuring in this way, the aqueous phase prepared in the aqueous phase preparation line WL and the oil phase prepared in the oil phase preparation line OL are sequentially put into the mixing tank 30, whereby diesel fuel of the present invention can be produced on-the-go, onsite, or both.

Still another embodiment of the system of the present invention will now be described with reference to FIG.5. Components similar to those in FIG. 3 and FIG.4 are denoted by the same reference numerals, and descriptions thereof are omitted.

In this embodiment, a large amount of water is used in the aqueous phase preparation line WL, and therefore the reformed water is used for various purposes, such as water for emergency measures in the event of a disaster, drinking water, domestic water, and agricultural water, etc. can be used in combination.

In this case, it is preferable to apply radicals to water in the reformer 11, amplify and maintain the applied radicals, and then scavenge the radicals once or repeatedly.

It has been found that when soft water is passed through an apparatus that repeats a radical generation process, a radical amplification/maintenance process, and a radical scavenging process, microbes in the water are killed, the permeability increases, and the REDOX potential decreases. When the first enzyme is added and matured in such water environments, the diffusion/maturation speed and activation of the first enzyme are increased, and the maturation time is shortened by about 5% to 10% and the sterilized soft water with low hardness can be used for the water of the aqueous phase preparation line WL of the present invention together with water for various usage.

Although the embodiments of the present invention have been described above, the present invention is not limited to these embodiments.

### INDUSTRIAL APPLICABILITY

According to the present invention, using a base oil made of light oil derived from Petro diesel as a starting material, diesel fuel can be obtained at about 22.5 to 45% by volume of the base oil, i.e., 1.225 to 1.45 times the total volume of the base oil, by reacting a water phase having an aqueous enzyme solution containing a lipase enzyme as a first enzyme admixed with a water-soluble alcohol to form a 20 to 35% aqueous alcohol solution with an oil phase having a pineapple-derived enzyme containing a second enzyme, bromelain, preliminarily added as a reaction accelerator.

The resulting diesel fuel conforms to the performance standards of each country, and has improved properties at least one property selected from the group consisting of a cetane number, a cetane index, a polycyclic aromatic content, a flash point, a sulfur content, carbon residue on 10% distillation residue, a pour point, ash weight and viscosity. More specifically, the cetane number (cetane index) and flash point are increased, and the aromatic content, kinematic viscosity, and sulfur content are decreased, resulting in a high-quality diesel fuel.

Even if the base oil which does not meet the standards of each country is used as a starting material, for example, even if a raw material with a cetane index of about 40 or a raw material with a sulfur content of about 0.5% by weight is used, the resulting diesel fuel meets the standards of each country.

Furthermore, when the resulting diesel fuel with improved properties is applied to a diesel engine, the amount of CO, SOₓ and PM generated decreases without increasing NOₓ in the exhaust gas. This means that while maintaining the advantages of diesel fuel, such as good fuel efficiency and the ability to obtain high torque, it overcomes the drawbacks that NOₓ and PM are easily emitted and that the exhaust gas is not clean. Such advantages can be attained by the present invention for the first time.

Furthermore, according to the present invention, there is provided a system for stably producing diesel fuel having improved properties conforming to the standards of each country.

### DESCRIPTION OF SYMBOLS

- WL: Water phase preparation line
- WS: Water source
- WT: Water phase tank
- OL: Oil phase reparation line
- OS: Base oil
- OT: Oil phase tank
- PL: Diesel fuel production line
- PT: Diesel fuel tank
- M: Mobile body
- PO: Pump for supplying oil phase
- PW: Pump for supplying water phase
- 10: Material tank
- 11: Water modification device
- 12: Enzyme mixing tank
- 12a: Device for adding first enzyme
- 13: Maturation tank (Tank for preparing aqueous enzyme solution)
- 14: First mixing tank (Tank for mixing water and alcohol)
- 14a: Alcohol tank
- 20: Base oil preparation tank (second mixing tank)
- 20a: Device for adding second enzyme
- 30: Oil/water mixing tank
- 31: Stirring Tank
- 32: Pulsing device
- 33: Reactor
- 34: Purification device

## Claims

1. A method for producing diesel fuel having improved properties at least one property selected from the group consisting of a cetane number, a cetane index, a polycyclic aromatic content, a flash point, a sulfur content, carbon residue on 10% distillation residue, a pour point, ash weight and viscosity using light oil derived from Petro diesel, comprising the following stages:
(a) a water phase preparation stage for preparing a water phase which comprises mixing an aqueous enzyme solution containing 0.01 to 0.1 % by weight of a lipase enzyme as a first enzyme with a water-soluble alcohol so as to be an aqueous alcohol solution having an alcohol concentration of from 20 to 35%;
(b) an oil phase preparation stage for preparing an oil phase which comprises adding a second enzyme solution in light oil having 0.001 to 0.01% by weight of pineapple-derived enzyme containing bromelain, dissolved in light oil to said base oil;
(c) a stage for mixing the oil phase with the water phase which comprises adding the water phase prepared in stage (a) to the oil phase prepared in stage (b) with stirring, while maintaining the temperature not exceeding 60°C, to be the volume ratio of the water phase to the oil phase to within the range of 2-4: 8-6;
(d) a reaction stage, which comprises leaving the oil/water mixture at normal room temperature while continuously or discontinuously contacting both phases with vibration or stirring to cause the reaction between the water phase and the oil phase to improve the properties of the oil phase and to produce diesel fuel; and
(e) a separation and refining stage, which comprises, after the reaction stage (d), separating the oil phase from the water phase, and refining the separated oil phase by precision filtration.

2. The method according to Claim 1, wherein the stage (c) for mixing the oil phase with the water phase comprises adding the water phase to the oil phase at a pressure of 2.5-3MPa and at a liquid temperature not exceeding 40°C.

3. The method according to Claim 1 or 2, wherein the stirring is conducted in vortex flow.

4. The method according to anyone of Claims 1 to 3, which further comprises, after the stage (c) for mixing the oil phase with the water phase, a stage for removing impurities which comprises applying said oil water mixture to a pulse wave or vibration to remove impurities from the oil phase

5. The method according to anyone of Claims 1 to 4, wherein the water phase is prepared by using soft water having a hardness in the range of 0-60mg/L as a water source of said aqueous enzyme solution, adding said first enzyme to the soft water, and maturing the enzyme at a temperature of from 20 to 30°C with aeration over a period of at least 60 hours to prepare the aqueous enzyme solution, and then adding the alcohol to the prepared aqueous enzyme solution.

6. The method according to Claim 3, wherein said soft water is modified by one or multiple set of treatment of radical treatment and radical scavenge treatment.

7. Diesel fuel having improved properties produced by the method according to anyone of Claims 1 to 6.

8. A system for producing diesel fuel having improved properties produced by the method according to anyone of Claims 1 to 6, comprising:
A1: an water phase line having a water phase tank which stores the water phase prepared by mixing the aqueous enzyme solution containing 0.01-0.10% by weight of lipase as the first enzyme with the water-soluble alcohol so as to be an aqueous alcohol solution having an alcohol concentration of from 20 to 35%, with aeration;
B1: a base oil tank which stores a light oil derived from Petro diesel;
B2: an oil phase line having an oil phase preparation member which prepares the oil phase by adding the second enzyme to said base oil;
C1: a oil/water mixing tank which meters the prepared oil phase, and added thereto the water phase at a prescribed pressure so that the temperature liquid is within a prescribed range;
C2: a reactor having a vibrator which vibrates the oil/water mixture from the oil/water mixing tank and/or a pulsing device which pulses the oil/water mixture and refining the separated diesel fuel;
C3: a separation/refining device which separates diesel fuel having improved properties obtained by the reaction from the water phase; and
C4: a diesel fuel production line having a storage tank which stores the diesel fuel having improved properties thus separated and purified.

9. The system according to Claim 8, which further possesses at least one reactor tank possessing or not possessing a vibrator and/or a pulsing device, on downstream of said reactor.

10. The system according to Claim 8 or 9, wherein said diesel fuel production line is accommodated within a mobile body.

11. The system according to Claim 9, wherein said water phase line further possesses an enzyme mixing tank which admixes raw water from water source with the lipase enzyme as the first enzyme, a maturation tank having an aeration device, which maturates the mixed solution of said raw water with the lipase enzyme with aeration to prepare the aqueous enzyme solution, and a mixing tank which mixes the aqueous enzyme solution thus prepared with the alcohol to prepare the water phase.

12. Use of pineapple-derived enzyme containing bromelain as a reaction accelerator for adding an oil phase, in the course of the production of diesel fuel by admixing a water phase comprising an aqueous alcohol solution containing lipase enzyme with the oil phase comprising Petoro diesel or crude Petro diesel.
